# EUROPÄISCHE PATENTSCHRIFT

(11) **EP 3 318 227 B1**
(45) Veröffentlichungstag und Bekanntmachung des Hinweises auf die Patenterteilung: **09.01.2019**
(21) Anmeldenummer: 16197287.2
(22) Anmeldetag: 04.11.2016
(51) Int. Cl.: A61F 9/008, A61F 9/007

(54) **SYSTEM FÜR DIE FRAGMENTIERUNG EINES AUGENLINSENKERNS**
SYSTEM FOR THE FRAGMENTATION OF AN EYE LENS CORE
SYSTÈME DE FRAGMENTATION D'UN NOYAU DU CRISTALLIN

(43) Veröffentlichungstag der Anmeldung: 09.05.2018
(73) Patentinhaber: IROC Services AG, 6300 Zug (CH)
(72) Erfinder: Seiler, Theo, 8002 Zürich (CH); Seiler, Theo Günther, 8002 Zürich (CH); Seiler, Stefan, 8002 Zürich (CH)
(74) Vertreter: Frenkel, Matthias Alexander

(56) Entgegenhaltungen:
- WO-A1-2016/168759
- DE-A1-102013 211 854
- DE-A1-102015 202 772
- US-A1- 2016 220 110
- Besner S. et al.: "In Vivo Brillouin Analysis of the Aging Crystalline Lens", Invest Ophthalmol Vis Sci, Bd. 57, Nr. 13 1. Oktober 2016 (2016-10-01), Seiten 5093-5100, XP002769479, Gefunden im Internet: URL:https://www.ncbi.nlm.nih.gov/pubmed/27 699407
- Stephan Reiss et al.: "Spatially resolved Brillouin spectroscopy to determine the rheological properties of the eye lens", Biomed Opt Express, Bd. 2, Nr. 8 1. August 2011 (2011-08-01), Seiten 2144-2159, XP002769480, Gefunden im Internet: URL:https://www.ncbi.nlm.nih.gov/pmc/artic les/PMC3149515/

## Beschreibung

### Hintergrund der Erfindung

Die Erfindung betrifft ein System für die Fragmentierung eines Augenlinsenkerns im Zusammenhang mit einer sogenannten Kateraktoperation (Operation des grauen Stars).

Eine Kateraktoperation besteht aus mehreren Schritten, unter anderem einer Zerkleinerung des Inhaltes der Augenlinse und dabei insbesondere des harten Kernes der Linse. Die menschliche Augenlinse hat keine Gefäßversorgung und deshalb werden lebenslang die Stoffwechselprodukte im Linsenkern abgelegt und dort verdichtet. Es entsteht ein Protein-Parakristall, welcher mit zunehmendem Alter härter wird. Im Stand der Technik wird bei der Kateraktchirurgie mittels einer Ultraschallsonde der Augenlinsenkern zertrümmert (fragmentiert) und die Fragmente werden abgesaugt. Dieser Vorgang kann erhebliche Zeit in Anspruch nehmen und ist auch eine Ursache für mögliche Komplikationen.

### Stand der Technik

In der jüngeren Vergangenheit hat zunehmend eine neue Technik der sogenannten Phakoemulsifikation Einzug gehalten, nämlich die Photofragmentierung und Photokapsulorhexis. Dabei wird ein Femtosekundenlaser (also ein Laser mit Pulslängen im Femtosekundenbereich) eingesetzt, um einerseits eine runde und definierte Eröffnung der vorderen Linsenkapsel zu ermöglichen und andererseits den Linsenkern in kleine Stücke zu fragmentieren, die dann, ähnlich wie bei der oben genannten herkömmlichen Technik, entfernt werden können, damit eine neue intraokulare Linse eingesetzt werden kann.

Bei der Photofragmentierung mit dem Femtosekundenlaser werden Fragmentierungen im harten Material des Augenlinsenkerns durchgeführt. Relevanter Stand der Technik findet sich in WO 2016/168759, DE 10 2015 202772, DE 10 2013 211854, Besner S. et al.: "In Vivo Brillouin Analysis of the Aging Crystalline Lens", Invest Ophthalmol Vis Sci, Stephan Reiss et al.: "Spatially resolved Brillouin spectroscopy to determine the rheological properties of the eye lens",Biomed Opt Express und US 2016/220110.

### Kurzbeschreibung der Erfindung

Ziel der Erfindung ist die Bereitstellung eines Systems für die Fragmentierung eines Augenlinsenkerns, mit dem eine Kataraktoperation vereinfacht und mit besseren Ergebnissen durchführbar ist.

Hierzu lehrt die Erfindung ein System für die Fragmentierung eines Augenlinsenkerns, aufweisend: eine erste Laserstrahlungsquelle, eingerichtet für eine Bestrahlung des Augenlinsenkerns mit für eine Brillouin-Spektrometrie geeigneter erster Strahlung; eine Vorrichtung zur Durchführung einer Brillouin-Spektrometrie mit von dem Augenlinsenkern rückgestreuter Strahlung zur Gewinnung von Brillouinstreuungsmessdaten; eine Recheneinheit, in der Korrelationen zwischen Brillouinstreuungsmessdaten und Parametern einer für die Fragmentierung eines Augenlinsenkerns geeigneten zweiten Strahlung enthalten sind oder abgeleitet werden; und eine zweite Laserstrahlungsquelle mit Strahlungsführungsmitteln, eingerichtet für eine Bestrahlung des Augenlinsenkerns mit der zweiten Strahlung mit den zu dem Brillouinstreuungsmessdaten des bestrahlten Augenlinsenkerns korrelierten Parametern zur Fragmentierung des Augenlinsenkerns.

Der Erfindung liegt die Erkenntnis zugrunde, dass es für eine Kataraktoperation hilfreich ist, Eigenschaften der für die Fragmentierung eingesetzten Pulse des Femtosekundenlasers an Eigenschaften des Augenlinsenkerns anzupassen. Augenlinsenkerne haben von Patient zu Patient unterschiedliche Eigenschaften hinsichtlich ihrer Härte und allgemein hinsichtlich ihrer Fähigkeit, mit Femtosekundenlaserpulsen durch Mikroexplosionen zertrümmert zu werden. Je härter der Augenlinsenkern, umso größer muss in der Regel die Energiedichte des Lasers am Ort der Mikroexplosion sein.

Weiterhin liegt der Erfindung die Erkenntnis zugrunde, dass die sogenannte Brillouin-Spektrometrie geeignet ist, hier relevante Eigenschaften des Augenlinsenkerns zu ermitteln. Bei der Brillouin-Spektrometrie handelt es sich um eine unelastische Streuung von Licht an akustischen Phononen. Bei der Streuung von Photonen im Bereich des sichtbaren Lichts in Kristallen (hier also dem Augenlinsenkern) tritt eine Emission oder Absorbtion von Phononen mit sehr kleinen Frequenzverschiebungen im gestreuten Licht auf. Es werden Stokes-Verschiebungen und Antistokes-Verschiebungen unterschieden. Die Techniken der Brillouin-Spektrometrie sind als solches weit entwickelt und auf diesen Stand der Technik kann hier zurückgegriffen werden.

Mit dem erfindungsgemäßen System kann mittels der Vorrichtung für die Durchführung einer Brillouin-Spektrometrie ("Brillouin-Spektrometer") präoperativ eine Information bezüglich Eigenschaften des Augenlinsenkerns gewonnen werden, die für eine nachfolgende operative Fragmentierung des Kerns Bedeutung haben. Mit anderen Worten: die Erfindung macht sich die Erkenntnis zu Nutze, dass eine Korrelation besteht zwischen Ergebnissen der Brillouin-Spektrometrie am Augenlinsenkern und Parametern (Eigenschaften) der Femtosekundenlaserpulse, die zu den besten Ergebnissen hinsichtlich der Fragmentierung führen. Insbesondere kann diese Korrelation experimentell/empirisch aufgestellt werden, zum Beispiel durch Auswertung empirischer Operationsdaten und/oder anhand von Vergleichsversuchen etc.

Solche empirisch ermittelten Korrelationen zwischen durch Brillouin-Spektrometrie ermittelten Eigenschaften des Augenlinsenkerns und Parametern der Femtosekundenlaserpulse, welche für diese Eigenschaften des Augenlinsenkerns (also beim konkreten Patienten) optimale Ergebnisse liefern, können zum Beispiel in Tabellenform in einem Rechner abgelegt werden, der zu dem System gehört. Dabei kann gegebenenfalls in die Korrelation der Parameter "Augenlinsenhärte" eingeschoben werden, d.h. zunächst wird aus den Daten der Brillouin-Spektrometrie eine Aussage über die Härte des Augenlinsenkerns abgeleitet und aus der Härte des Augenlinsenkerns wird der optimale Parametersatz für die Femtosekundenpulse abgeleitet (wiederum auf Basis von empirisch ermittelten Zusammenhängen diesbezüglich).

Als Parameter der Femtosekundenlaserpulse kommen insbesondere in Betracht: Pulslänge, Pulsfrequenz, Puls-Energie, Puls-Energiedichte, Wellenlänge, und/oder die Puls-Abmessungen am Wirkungsort im Augenlinsenkern.

Femtosekundenlaserpulse (hier mit "zweite Strahlung" bezeichnet) werden so in den Augenlinsenkern geführt, dass sie am gewünschten Ort der Mikroexplosion ihre höchste Dichte haben. Dies kann zum Beispiel durch stark fokussierende optische Mittel erfolgen derart, dass der Fokus am gewünschten Ort der Mikroexplosion liegt.

Bevorzugt werden die Femtosekundenlaserpulse mit mehreren seitlich zueinander versetzten (z.B. parallelen) Linien auf dem Augenlinsenkern abgebildet, so dass mehrere nebeneinanderliegende Schnitte im Augenlinsenkern erzeugt werden, um ihn zu fragmentieren. Die Schnitte werden bevorzugt kreuzweise angebracht, d.h. in im Wesentlichen einem Gittermuster. Dabei wird bevorzugt das Muster und/oder die Energie der Strahlung in Abhängigkeit von der Härte des Augenlinsenkerns derart eingestellt, dass mit härter werdendem Augenlinsenkern der Abstand zwischen den Linien (also auch den Schnitten) größer wird und die Energie pro Linie (Schnitt) ebenfalls größer wird im Vergleich zu weniger harten Augenlinsenkernen. Empirisch hat sich dies als vorteilhaft erwiesen. Auch hier wird die Korrelation zwischen den Ergebnissen der Brillouin-Spektrometrie und der optimalen Gestaltung der Schneidlinien empirisch ermittelt und im System abgelegt für einen optimalen Einsatz des Systems in Abhängigkeit von den Eigenschaften des Augenlinsenkerns des Patienten.

Andererseits können aber die Strahlungsführungsmittel auch optische Fasern aufweisen, die eingerichtet sind, zum Augenlinsenkern geschoben zu werden, um dort die Mikroexplosion auszulösen.

Die Techniken der Brillouin-Spektrometrie einerseits und die Techniken der Fragmentierung mit Femtosekundenlaserpulsen andererseits, sind jeweils als solche bekannt und darauf kann bei Ausführung der vorliegenden Erfindung zurückgegriffen werden.

Die Erfindung liefert also eine Anpassung der Explosionsenergie bei den Mikroexplosionen an die Eigenschaften des Augenlinsenkerns mittels Einstellung von Parametern der Femtosekundenlaserpulse, wie Pulsenergie/Schnittweite.

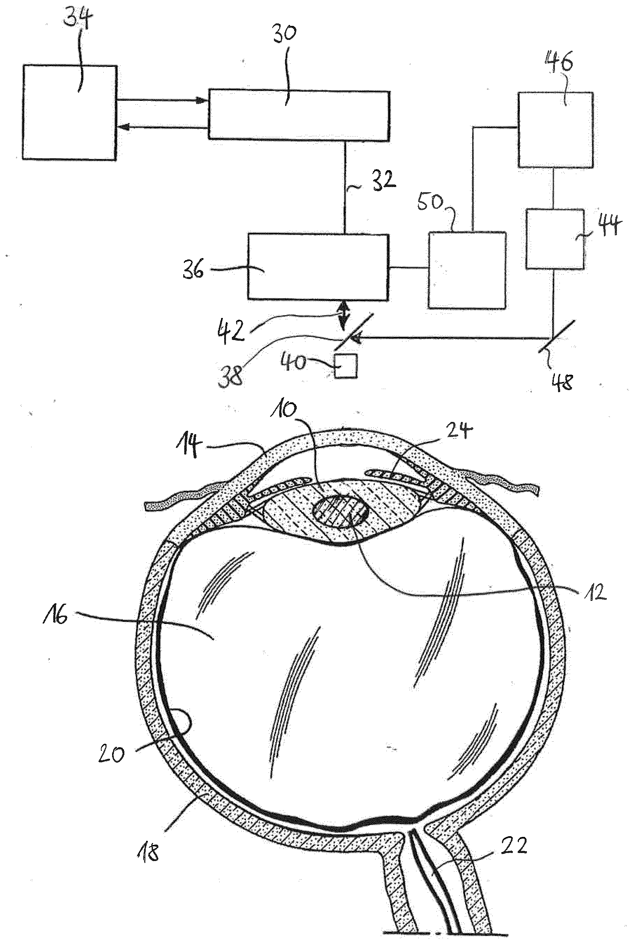

Nachfolgend wird ein Ausführungsbeispiel der Erfindung mit Bezug auf die Figur näher beschrieben.

Die Figur zeigt schematisch ein System für die Fragmentierung eines Augenlinsenkerns.

Die Figur zeigt eine Augenlinse 10 mit Augenlinsenkern 12, die Hornhaut (Kornea) 14, Glaskörper 16, Lederhaut (Sklera) 18, Netzhaut (Retina) 20, Sehnerv 22 und Iris 24.

Eine erste Strahlungsquelle 30 wird für die Brillouin-Spektrometrie eingesetzt. Sie erzeugt eine hierfür geeignete erste Laserstrahlung, typischerweise im sichtbaren Bereich. Eine Steuerung 34 steuert die Strahlungsquelle 30. Die "erste" Strahlung 32 der Strahlungsquelle 30 wird mit als solches bekannten Mitteln auf und in den Augenlinsenkern 12 gerichtet und vom Augenlinsenkern 12 rückgestreute Strahlung wird mit dem Brillouin-Spektrometer 36 vermessen. Der Doppelpfeil 42 deutet die Richtung der Messstrahlung und der rückgestreuten Strahlung an.

Die Messdaten des Brillouin-Spektrometers 36 werden in einen Rechner 50 eingegeben.

Im Rechner 50 sind zum Beispiel Daten in Tabellenform bezüglich einer Korrelation zwischen den Messdaten des Brillouin-Spektrometers 36 und Parametern von Femtosekundenlaserpulsen abgelegt, welche Daten empirisch gewonnen worden sind und welche zu bei einem bestimmten Patienten ermittelten Messdaten des Brillouin-Spektrometers 36 optimale Parameter für Femtosekundenlaserpulse angeben, mit welchem eine gute Fragmentierung des Augenlinsenkerns 12 erreicht wird.

Femtosekundenlaserpulse mit diesen Parametern werden dann mittels einer zweiten Strahlungsquelle 44, also einem Femtosekundenlaser, mit der Steuerung 46 erzeugt. Die so erzeugten Femtosekundenlaserpulse werden dann über schematisch dargestellte Spiegel 38, 48 etc. so in den Augenlinsenkern 12 gerichtet, dass dort an gewünschten Stellen die höchste Strahlungsdichte zur Erzeugung der genannten Mikroexplosionen gegeben ist. Die Spiegel 38, 48 sowie die Strahlungsführungsmittel 40 sind der Einfachheit halber für die erste und die zweite Strahlung gleich dargestellt, jedoch sind in der Praxis für die erste Strahlung und die zweite Strahlung jeweils optimale Strahlungsführungsmittel in den Strahlengang einführbar.

Die Steuerungen 34, 46 für die Laser 30 beziehungsweise 44 sowie der Rechner 50 können in einer einzigen Recheneinheit integriert sein.

### Bezugszeichen

- 10: Augenlinse
- 12: Augenlinsenkern
- 14: Hornhaut (Kornea)
- 16: Glaskörper
- 18: Lederhaut (Sklera)
- 20: Netzhaut (Retina)
- 22: Sehnerv
- 24: Iris
- 30: erste Strahlungsquelle
- 32: erste Strahlung
- 34: Steuerung (für 30)
- 36: Brillouin-Spektrometer
- 38: Spiegel
- 40: Strahlführungsmittel
- 42: Doppelpfeil
- 44: zweite Strahlungsquelle
- 46: Steuerung (für 44)
- 48: Spiegel
- 50: Rechnereinheit
- 52: zweite Strahlung

## Patentansprüche

1. System für die Fragmentierung eines Augenlinsenkerns (12), aufweisend:
- eine erste Laserstrahlungsquelle (30), eingerichtet für eine Bestrahlung des Augenlinsenkerns (12) mit für eine Brillouin-Spektrometrie geeigneter erster Strahlung (32);
- eine Vorrichtung (36) eingerichtet für die Durchführung einer Brillouin-Spektrometrie mit von dem Augenlinsenkern (12) rückgestreuter Strahlung zur Gewinnung von Brillouinstreuungsmessdaten;
- eine Recheneinheit (50), in der Korrelationen zwischen Brillouinstreuungsmessdaten und Parametern einer für die Fragmentierung des Augenlinsenkerns (12) geeigneten zweiten Strahlung (52) enthalten sind oder abgeleitet werden; und
- eine zweite Laserstrahlungsquelle (44) mit Strahlungsführungsmitteln (40), eingerichtet für eine Bestrahlung des Augenlinsenkerns (12) mit der zweiten Strahlung mit den zu dem Brillouinstreuungsmessdaten des bestrahlten Augenlinsenkerns (12) korrelierten Parametern zur Fragmentierung des Augenlinsenkerns.

2. System nach Anspruch 1, **dadurch gekennzeichnet, dass** die zweite Strahlungsquelle (44) ein Femtosekundenlaser ist.

3. System nach einem der Ansprüche 1 oder 2, **dadurch gekennzeichnet, dass** die Parameter der zweiten Strahlung insbesondere einer oder mehrere der folgenden sind: Pulslänge, Pulsfrequenz, Puls-Energie, Puls-Energiedichte, Wellenlänge, Puls-Abmessungen am Wirkungsort im Augenlinsenkern (12).

4. System nach einem der vorhergehenden Ansprüche, **dadurch gekennzeichnet, dass** die Strahlungsführungsmittel (40) fokussierende optische Mittel sind.

5. System nach einem der Ansprüche 1 bis 3, **dadurch gekennzeichnet, dass** die Strahlungsführungsmittel (40) optische Fasern aufweisen, die eingerichtet sind, zum Augenlinsenkern geschoben zu werden.

6. System nach einem der vorhergehenden Ansprüche, **dadurch gekennzeichnet, dass** die zweite Strahlung (52) linienförmig zur Erzeugung von linienförmigen Schnitten auf den Augenlinsenkern gerichtet wird.

7. System nach Anspruch 6, wobei die zweite Strahlung gemäß einem Muster von mehreren zueinander versetzten Linien auf den Augenlinsenkern gerichtet wird.

8. System nach einem der Ansprüche 6 oder 7, wobei das Muster der Linien gitterförmig ist.

9. System nach einem der Ansprüche 6 bis 8, wobei mit zunehmender Härte des Augenlinsenkerns der Abstand der Linien und die Energie der zweiten Laserstrahlung pro Linie vergrößert werden im Vergleich zu einem Augenlinsenkern geringerer Härte.

## Claims

1. A system for fragmenting an eye lens nucleus (12), comprising:
- a first laser radiation source (30), configured to irradiate the eye lens nucleus (12) with first radiation suitable for Brillouin spectroscopy;
- an apparatus (36) configured for performing Brillouin spectroscopy with radiation scattered back from the eye lens nucleus (12) in order to acquire Brillouin scattering measured data;
- a processing unit (50) in or from which correlations between Brillouin scattering measured data and parameters of a second radiation (52) suitable for fragmenting the eye lens nucleus (12) are contained or derived; and
- a second laser radiation source (44) having radiation guiding means (40), configured to irradiate the eye lens nucleus (12) with the second radiation with the parameters correlated with the Brillouin scattering measured data of the irradiated eye lens nucleus (12), in order to fragment the eye lens nucleus.

2. The system as claimed in claim 1, **characterized in that** the second radiation source (44) is a femtosecond laser.

3. The system as claimed in claim 1 or 2, **characterized in that** the parameters of the second radiation are in particular one or more of the following: pulse length, pulse frequency, pulse energy, pulse energy density, wavelength, pulse dimensions at the site of action in the eye lens nucleus (12).

4. The system as claimed in any one of the foregoing claims, **characterized in that** the radiation guiding means (40) are focusing optical means.

5. The system as claimed in any one of claims 1 to 3, **characterized in that** the radiation guiding means (40) have optical fibers which are configured to be pushed to the eye lens nucleus.

6. The system as claimed in any one of the foregoing claims, **characterized in that** the second radiation (52) is directed linearly on the eye lens nucleus in order to produce linear cuts.

7. The system as claimed in claim 6, wherein the second radiation is directed at the eye lens nucleus in a pattern of a plurality of mutually offset lines.

8. The system as claimed in claim 6 or 7, wherein the pattern of lines is grid-like.

9. The system as claimed in any one of claims 6 to 8, wherein, as the hardness of the eye lens nucleus increases, the spacing of the lines and the energy of the second laser radiation per line are increased compared with an eye lens nucleus of lower hardness.

## Revendications

1. Système pour la fragmentation d'un noyau du cristallin (12) comprenant :
- une première source de rayonnement laser (30) conçue pour irradier le noyau du cristallin (12) avec un premier rayonnement (32) approprié à la spectrométrie Brillouin ;
- un dispositif (36) conçu pour effectuer une spectrométrie Brillouin avec un rayonnement rétrodiffusé par le noyau du cristallin (12) pour obtenir des données de mesure de diffusion Brillouin ;
- une unité de calcul (50) dans laquelle sont contenues ou dérivées des corrélations entre des données de mesure de diffusion Brillouin et des paramètres d'un second rayonnement (52) approprié à la fragmentation du noyau du cristallin (12) ; et
- une seconde source de rayonnement laser (44) dotée de moyens de guidage de rayonnement (40), conçue pour irradier le noyau du cristallin (12) avec le second rayonnement avec les paramètres corrélés aux données de mesure de diffusion Brillouin du noyau du cristallin (12) irradié pour fragmenter le noyau du cristallin (12).

2. Système selon la revendication 1, **caractérisé en ce que** la seconde source de rayonnement (44) est un laser femtoseconde.

3. Système selon l'une des revendications 1 ou 2, **caractérisé en ce que** les paramètres du second rayonnement sont en particulier un ou plusieurs des paramètres suivants : longueur d'impulsion, fréquence d'impulsion, énergie d'impulsion, densité d'énergie d'impulsion, longueur d'onde, dimensions d'impulsion sur le site d'action dans le noyau du cristallin (12).

4. Système selon l'une des revendications précédentes, **caractérisé en ce que** les moyens de guidage de rayonnement (40) sont des moyens optiques de focalisation.

5. Système selon l'une des revendications 1 à 3, **caractérisé en ce que** les moyens de guidage de rayonnement (40) comprennent des fibres optiques qui sont conçues pour être poussées vers le noyau du cristallin.

6. Système selon l'une des revendications précédentes, **caractérisé en ce que** le second rayonnement (52) est dirigé linéairement pour produire des coupes linéaires sur le noyau du cristallin.

7. Système selon la revendication 6, dans lequel le second rayonnement est dirigé sur le noyau du cristallin selon un motif de plusieurs lignes mutuellement décalées.

8. Système selon l'une des revendications 6 ou 7, dans lequel le motif des lignes est en forme de grille.

9. Système selon l'une des revendications 6 à 8, dans lequel, avec une dureté du noyau du cristallin croissante, la distance entre les lignes et l'énergie du second rayonnement laser par ligne sont augmentées par rapport à un noyau du cristallin de dureté moindre.
